# EUROPEAN PATENT APPLICATION

(11) **EP 1 057 401 A1**
(43) Date of publication of application: **06.12.2000**
(21) Application number: 99201787.1
(22) Date of filing: 04.06.1999
(51) Int. Cl.: A01H 1/02, A01H 5/10

(54) **Seedless tomato and method for producing a seedless tomato, hybrid tomato plants capable of producing said seedless tomatoes and cultivation material therefore, and food products obtained from said seedless tomatoes**

(71) Applicant: Western Seed Espana S.A., Bajada de Vargas 35080 Carrizal-Ingenio (ES)
(72) Inventor: Van Vliet, Gerardus, J., A., 35120 Pasito Blanco Las Palmas (ES)
(74) Representative: de Bruijn, Leendert C.

(57) **Abstract**

The invention relates to a method for producing a seedless tomato, a plant carrying seedless tomatoes or capable of carrying seedless tomatoes, or cultivation material for such a tomato plant such as seed, comprising the steps of:
a. providing a first tomato plant that contains the *pk,fs*-complex;
b. providing a second tomato plant that contains the *pk,fs*-complex;
c. crossing the first and second tomato plant for the production of cultivation material, such as seed, which contains the *pk,fs*-complex;
d. optionally cultivating the cultivation material thus obtained into a tomato plant capable of carrying seedless tomatoes;
e. optionally growing said tomato plant until it carries the seedless tomatoes, and harvesting the seedless tomatoes thus obtained.

The invention also relates to a hybrid tomato plant capable of carrying seedless tomatoes, obtained via said method, to seed or cultivating material for such a hybrid tomato plant, to a seedless tomato, obtained as fruit from such a hybrid tomato plant. Furthermore, the invention relates to processed products, in particular processed food products, obtained from or using, or containing, such a seedless tomato.

## Description

### Introduction:

The present application relates to a seedless tomato, a method for obtaining said seedless tomato, as well as a method for processing the seedless tomato thus obtained to products, in particular food products.

WO 98/24301 in the name of applicant describes a seedless tomato and a method for producing said tomato, in which a first tomato plant that is homozygote recessive with regard to the properties parthenocarpy (pk,pk) and functional sterility (fs,fs) is crossed with a second tomato plant that is also homozygote recessive with regard to the properties parthenocarpy (pk,pk) and functional sterility (fs,fs).

The present invention represents an improvement over the teaching of WO 98/24301, in that it provides hybrids (and seed therefor) that allow for reliable production of seedless tomatoes under all enviromental conditions, including different light and temperature conditions as may be prevalent in both the tropics as well as moderate climates. This is necessary in order to provide hybrids and seed therefor that can be succesfully commericalized and grown in all countries of the world, including the emerging tomato growing countries such as those in the Far East.

H.Georgiev et al., Eucarpia Tomato-90, Proceedings of the XI Eucarpia Meeting on Tomato Genetics and Breeding, Malaga, Spain, March 1990: "Breeding of Seedless Tomatoes" describe a method for obtaining tomato plants that carry completely seedless tomato fruits, by combining in one cultivar the homozygous genes for parthenocarpy *pat-2* and the homozygous gene for autosterility of flowers *ps-2.* The *pat-2, ps-2* cultivar thus obtained carries completely seedless tomato fruits (as shown in Tables 1 and 2 of the Georgiev reference). Georgiev et al. further describe that by crossing two such cultivars, F1 hybrids can be created that carry completely seedless and standard fruits.

However, extensive research by applicant into the line(s) described by Georgiev has shown that when the property of parthenocarpy and the property of autosterility are combined to provide parent lines, and hybrid seed is obtained from two of these parent lines - i.e. by means of human intervention as described below - that the presence of only the double recessive *pat-2* gene and only the double recessive *ps-2* gene is in practice not sufficient to provide hybrids that will stably and reliably form seedless tomatoes under all growing conditions. Therefore, crossing two parent lines that both contain only the double recessive genes *(pat-2, pat-2)* and *(ps-2, ps-2)* will not lead to commercially acceptable seedless hybrids, as the plants will not always, and not under all circumstances, provide well-formed tomatoes.

Acording to the present invention, it has been found that in order to provide hybrids that produce seedless tomatoes in a stable and reliable manner under all environmental conditions, a complex of genetic factors for both partenocarpy and for autosterility must be present in the parent plants or lines. These complexes are referred to herein as the "*pk*-complex" and the "*fs*-complex", respectively.

Applicant has also developed a general method for obtaining parent plants or lines that contain this *pk*-complex and *fs*-complex, starting from known lines that only contain the double recessive *pat-2* gene or the double recessive *ps-2* gene, or starting from the *pat-2, ps-2* line developed by Georgiev, above.

One step of this method may involve crossing a known seedless line such as the *pat-2, ps-2* line of Georgiev with a non-seedless tomato plant known per se, in order to provide a non-seedless F1. From this F1, after several further generations, parent plants or lines may then be developed in which the *pk*-complex and *fs*-complex have become sufficiently "fixated" for these plants to be used as parent lines for the production of hybrid seed.

In doing so, applicant has found that in generations derived from this F1, the inheritance of the seedless fenotype (and even of the phenotype of functional sterility) does not conform to what is expected on the basis of Mendelian principles. In particular, applicant has surprisingly found that, even when a tomato line with the seedless phenotype due to the presence of double recessive *pat-2* and *ps-2* genes (such as the line described by Georgiev) is used as starting material, in the F2 obtained by self-pollination of the F1 thus obtained, seedless plants are in fact very uncommon (i.e. limited to about 1 or 2 plants out of 100 F2 plants) or may even not occur at all, and may then not even occur when the functionally sterile plants from the F2 thus obtained are self-pollinated to provide an F3.

This is surprising because, on the basis of the fact that in the original *pat-2, ps-2* parent, the presence of the double recessive *pat-2* and *ps-2* genotype is sufficient to provide the desired seedless fenotype, it was to be expected on the basis of Mendelian rules that at least 1 out of 16 plants of said F2 (and even more plants in the subsequent F3) would have the double recessive *pat-2, ps-2* genotype and therefore show the seedless fenotype. In practice, much less or even no seedless plants are found in the F2 (and F3).

Nevertheless, despite the fact that said F2 shows less plants with the desired seedless fenotype than was expected - or in other words, despite the fact that the cross of the *pat-2, ps-2* line with a non-seedless tomato line apparently introduces genes, alleles and/or other genetic factors that reduce or suppress the desired property of seedlessness in said F2, or at least the full expression of the *pat-2* and/or *ps-2* genes - it has been found that starting from such an F2, seedless parent lines can be developed that provide seedless hybrids that are superior to those that can obtained using the known seedless parent lines with the *pat-2, ps-2* genotype.

The above (also) demonstrates the basic finding of the invention, i.e. that in order to stably and reliably provide hybrids that give seedless tomatoes under all environmental conditions, the presence of the *pat-2, ps-2* genotype by itself is not sufficient, but that in fact a complex of genes, alleles or other genetic factors for both partenocarpy and for auto-sterility must be present.

Besides this, applicant has also found that some non-seedless tomato plants or lines, when crossed with a known seedless line, are more likely to provide satisfactory seedless parent lines than others, and in greater numbers. The present description will therefore also teach the skilled person how to select from all tomato lines known per se - by means of a simple procedure - those plants or lines that can be used succesfully to provide parent lines for the production of seedless hybrids. The present description will also provide the skilled person with some guidance on selecting such non-seedless lines, based upon the experience gained by applicant in the course of his research.

The invention therefore provides a method for producing tomato plants that stably and reliably produce seedless tomatoes under all environmental, climatic and/or growing conditions, as well as seed for such tomato plants, by crossing two parent tomato plants or lines which carry both the *"pk* complex" and the *"fs* complex", as further described below. These two parent plants are referred to herein as the *"pk,fs-*parents".

### General description of the backround art:

In modern agriculture, tomatoes are most often grown by the grower/producer on hybrid tomato plants grown from hybrid tomato seed. The production of such hybrid seed is usually not carried out by the producer himself, but by highly specialized firms of plant breeders who develop and commerialize such hybrid seed, and who through stringent quality control can assure the uniform quality of the seed.

As a general rule, hybrid seed is obtained by crossing two different parent tomato plants, which most often belong to different lines. Using cultivation techniques and plant breeding techniques known per se, such hybrids can be provided with highly specific, desired properties, which makes it possible to "design" the hybrids, i.e. to confer to the hybrid plants predetermined inheritable characteristics.

This is usually achieved by suitably choosing (the properties of) the two parent lines which are crossed to provide the hybrid seed. These are usually inbred lines, obtained by self-fertilization (self-pollination) over several generations, and such inbred lines will usually again have been specifically "designed" by the breeder so as to provide hybrid offspring with the desired properties, when crossed with another - usually predetermined - inbred parent line.

As a rule, such parent lines will be genetically homozygote and identical (i.e. as a result of inbreeding) so that they can provide, in a stable and reliable manner, genetically uniform -albeit heterozygote- hybrid line combinations, which (can) combine the properties of the parent lines. In doing so, the aim is on the one hand to cross certain properties from the parent lines as purely as possible into the seed, while on the other hand use is made of the known effect of heterosis or inbred growth, which can provide (improved) properties regarding -inter alia- the growth of plants and fruits and thereby of the yield. This heterosis effect is obtained when/because the parent lines used are not related with respect to certain genetic properties (i.e. when the parent lines genetically "lie far apart").

For a further description of plant breeding techniques in general, and tomatoes in particular, using classical cultivation techniques, including the formation of hybrids, reference is made to the known handbooks, the contents of which are incorporated herein by reference.

Also, in this description, unless indicated otherwise, the terms and definitions used herein are those used in (Mendelian) genetics, for which reference is made to M.W. Strickberger, Genetics, second Edition (1976), in particular pages 113-122 and 164-177. As mentioned therein, *"gene"* generally means an inherited factor that determines a biological characteristic of an organism (i.c. a tomato plant), an *"allele"* is an individual gene in the gene pair present in the (diploid) tomato plant. A plant is called *"homozygous"* for a gene when it contains the same alleles of said gene, and *"heterozygous"* for a gene when it contains two different alleles of said gene. The use of capital letters indicates a dominant (form of a) gene and the use of small letters denotes a recessive gene: "*X,X*" therefore denotes a homozygote dominant genotype for gene or property X; "*X*,*x"* and "*x,X*" denote heterozygote genotypes; and "*x,x*" denotes a homozygote recessive genotype. As commonly known, only the homozygote recessive genotype will generally provide the corresponding recessive phenotype (i.e. lead to a plant that shows the property or trait "x") whereas the heterozygotic and homozygote dominant genotypes will generally provide the corresponding dominant phenotype (i.e. lead to a plant that shows the property or trait "X"), unless other genes and/or factors such as multiple alleles, suppressors, codominance etc. (also) play a role in determining the phenotype.

The tomatoes that are currently on the market have the disadvantage that they contain seeds (pips). The presence of these seeds for many consumers detracts from the attractiveness of the tomato. Also, in the preparation of a large number of products on the basis of tomatoes, in particular food products on the basis of tomatoes, the seeds have to be removed, for instance by sieving, optionally after prior pureeing, boiling or mashing, which involves further processing steps. This is true for both the preparation of food products on industrial scale, such as purée, soups, juices or sauces on tomato basis, as for the household preparation of dishes or food products.

Because of this, over the last 15-20 years, several investigators and tomato breeders have tried to develop tomato plants that produce seedless tomatoes. However, despite their efforts, which are discussed in the references mentioned in this application, no commercially acceptable seedless tomatoes, tomato plants or hybrids that grow such seedless tomatoes, or seed therefor, have so far reached the market.

The tomato flower consists of an ovary, above which there is a pistil. Around the pistil there are several stamen, that produce pollen. In the ovary, there are several pre-embryo's/embryo's which develop (after pollination with the pollen) into seeds.

The tomato plant can be considered as an "obligatory self-pollinator", which means that almost exclusively only its own pollen ends up on the stamen of the flowers of the same plant and thereby pollinates the pre-embryo's. As soon as pollinated pre-embryo's are formed in the ovary, the ovary starts to grow into a tomato (fruit) containing within it, at the same time, the developing seed.

In principle, fruits will only be formed when pollinated seeds are developing in the ovary; if the embryo's or pre-embryo's are not pollinated, no fruit will develop.

In tomato, a gene is known, called the *pat-2* (also sometimes referred to as *path-2)* gene, which codes for the property of parthenocarpy. This gene, when present as a double recessive *(pat-2, pat-2)* leads to developments of fruit (fruit flesh) without concomitant development of seed. In other words, a parthenocarpic plant may develop fruits even without pollination.

However, in nature, or in the greenhouse, (the phenotype of) parthenocarpy will usually only be partial; the factors which are responsible for the absence or partial presence of seeds are present on alleles. In partial parthenocarpy, seeds are or may be formed in (another) part of the fruit, with the result that the fruit will develop or grow irregularly. Partial parthenocarpy therefore leads to irregular forms of the fruit, and the resulting deformed fruits are highly unattractive for the consumer.

The gene of partenocarpy is extremely rare in nature. This is because, when a fruit is formed on the basis of total parthenocarpy, no seed is developed within the fruit, so that the genes coding for parthenocarpy are not passed on to the next generation, and the generational line is ended. For the tomato breeder, the phenotype of total parthenocarpy leads to fruits without seed, which makes seed production impossible.

The property of parthenocarpy resides in several alleles. The phenotype of total parthenocarpy can only occur when the "mother" (i.e. the tomato plant, the flower of which is pollinated with pollen) as well as the "father" (i.e. the tomato plant that provides the pollen) both are genetically homozygous with regard to the parthenocarpic property. This is because, in the fruit *"in statu nascendi",* there are usually several developing seeds, at least one of which may not become homozygote recessive for partenocarpy if one of the parents is a heterozygote.

Partial parthenocarpy (in the developing tomato, as the result of at least one seed which is not double recessive with regard to parthenocarpy) again may cause the fruit to develop in a deformed manner.

Another major problem with parthenocarpy, i.e. besides low seed yield and very uneven fruits in size and form within the clusters or within the plants due to partial presence or non-presence of seed in the fruits, is a strong dependence on the environment (temperature, light conditions).

For the above reasons, neither plants that are partenocarp to a very high degree, nor plants that (may) give rise to partial partenocarpy, can be used for the reliable production of seedless tomatoes.

Besides partenocarpy, another property that can occur in tomato is that of functional sterility (indicated herein as "FS"). A double recessive plant with regard to FS ("fs,fs") leads to a tomato plant with a pollen tube that is totally closed, so that the complete and fertile pollen cannot leave the pollen tube, not even by vibrating or other mechanical influences (bumble bees, insects or a vibrator).

The pollen of a tomato plant which has the double recessive ("fs,fs") phenotype can only be released by physically opening the pollen tube by hand (by cutting or scissoring), after which - in practice - the pollen has to be removed by hand from the opened pollen tube, i.e. by scraping. For fertilization of the same or another tomato plant, the pollen then has to be applied to the pistil of the flower, which in practice also must be carried out manually.

In any other "natural" way (i.e. without the abovementioned human intervention), the pollen of a functional sterile flower is not released and therefore not available for fertilization of a pre-embryo. A double recessive, functionally sterile plant therefore does not fertilize pre-embryo's, thereby ending the generational line so that the recessive genes for functional sterility are not passed on to the next generation. In nature, with a double recessive phenotype for functional sterility, no fertilization of the pre-embryo's will take place so that no fruit (tomato) will be formed. It has been suggested that the role of such autosterility genes in nature may be to prevent self-pollination and thereby to help maintain heterozygosity in plant populations through enforced disassortive mating.

Functional sterility is considered a form of a more general property of that occurs in tomato called auto-sterility, which can occur in two types, i.e:
- male sterile: self pollination is not possible because of the absence of viable pollen (ms) or degenerated stamens (sl, stamenless). When male sterility were to be introduced in a commercial seed hybrid tomato (seed), the grower has to sow double the amount of seed and to remove before planting the 50% of heterozygous ms pk plants, recognizable by a marker gene for ms. This is not fully possible by overcrossing problems.
- functional sterile; viable pollen is present but cannot reach the pistil due to some morphological deviation of the flowers. Functional sterility (fs) itself can also be distinguished in four different types, i.e.:
   - ps-type: an exerted style phenomenon as a result of strong twisting and shorting of the stamens; this property generally provides for easy self pollination and lower receptivity of the style, which makes it not very suitable for hybrid seed production.
   - ps-2-type : a non opening anther bags type succesfully used in hybrid seed production.
   - ex-type : exerted style over the stamens easy self pollination and low receptivity of the sigma make it less suitable for hybrid seed production.
   - short style-type: the stigma is located below the anthers, the main disadvantage is its high level of self pollination.

Both parthenocarpy and functional sterility (in particular of the ps-2 type) in tomato, as well as the genetic basis thereof, have been investigated in the prior art.

S. Lin, in Dissertation Abstracts International, Vol. 42, no.9, 1982, page 3514B, describes that the parthenocarpy of the tomato line *"Severianin"* is controlled by a single recessive gene, called *pat-2.* Lin further describes that the cross of *"Severianin x Positional Sterile ("Pat-Ex")"* provides a parthenocarpy-positional sterile-stigma exsertion *(Pat-Ex)* line, that can be used as a female line in F1 hybrid seed production without emasculation. Lin also describes that the F1 of the cross between *"Severianin"* and the *"Pat-Ex"* line (homozygous *pat-2/pat-2)* retains its stability to set parthenocarpic fruits.

In two separate papers from Session II - Male Sterility and Parthenocarpy, of the IXth Meeting of the Eucarpia Tomato Working Group, May 1984, J. Philouze and Ch. Georgiev et al., respectively, describe parthenocarpic tomato plants carrying the gene *pat-2,* as found in and derived from the line *"Severianin".*

In a separate paper from the same meeting, Hr.Geordiev and B.Atanassova describe the use of *a ps-2* sterile line with short style in the production of hybrid tomato seed.

Ch. Georgiev, in Genetika I. Selektsiya, Vol.18, no. 3, 1985, pages 264-266 describes that parthenocarpy was discovered in the course of transferring *ps-2* sterility in the orange fruited CV-Carobeta. This reference states that *"It was found that parthenocarpy is hereditary and is controlled by a single recessive gene. Tests of allelism with the known parthenocarpy genes are in progess".*

J. Philouze, Agronomie, Vol.9, no. 1, 1989, pages 63-75 describes a tomato line called *"75/59",* derived from a cross between the lines *"Atom"* and *"Bubjekosoko",* which line shows an aptitude for high level, natural but facultative parthenocarpy at all times of the year. Philouze further describes that the parthenocarpy of *"75/59"* is recessive, and that allelism tests show that the partenocarpy is not due to either the *pat-2* gene from *"Severianin"* or to the *pat* gene from *"Montfavet 191",* but is controlled by 3 recessive genes at least, and more probably by 4 or 5 genes, which are independant *of pat-2* and are speculated to act independently and cumulatively. Nevertheless, according to Philouze, seedlessness can be obtained by using *pat-2* alone, as in *Severianin.*

Philouze also uses the line *"75/59"* to produce seedless tomatoes, by a treatment involving emasculation without pollination. The appearance and weight of the seedless fruits thus obtained were comparable to those of the seeded fruits obtained by hand-pollination of the flowers of this line.

Nuez et al., Zeitschrift für Pflanzenzüchtung, Vol.96, no. 3, 1986, pages 200-206 describe a study of parthenocarpy in three tomato varieties: *"Sub-Arctic Plenty", "Severianin",* and the line *"59/75",* developed by Philouze, in 1 and F2 generations and in progenies from backcrosses to the parent. The crosses thus obtained were able to provide seedless fruits after emasculation.

In doing so, Nuez et al. state: *"Nevertheless, there is a lack of information about the genetic system for parthenocarpy in some varieties. Gene expression for parthenocarpy is strongly conditioned by environment and genetic background. Nothing has been quite established as to how such factors interact, what is the mode of genic action under some determined conditions, or how different genes juxtapose their effects to produce parthenocarpic answers.*"

From their study, Nuez et al. again conclude that the genes responsible for parthenocarpy on *"75/59"* and *"S.A.Plenty"* differ from *pat-2* from *'Severianin';* and the genes from *"75/59"* and *"S.A.Plenty"* are denominated as *pat-3, pat-4 and pat-5,* respectively.

However, the above references only describe tomato plants that have either a partenocarpic phenotype or a phenotype of auto-sterility. Also, based upon the natural variety *"Severianin",* the above art suggests that the presence of double recessive *pat-2* by itself is sufficient to cause parthenocarpy, as is the case in *Severianin* and the seedless *pat-2, ps-2* line described by Georgiev et al. in the Eucarpia Tomato reference. None of the above references describes plants that are homozygous recessive with respect to both the "*fs*-complex and "*pk*-complex"as defined herein, nor how such lines can be obtained.

### Brief description of the invention.

It has now been found that tomatoes without seeds/pips can be produced with advantage using -very preferably hybrid- tomato plants that combine a complex of - most probably recessive - genes encoding the phenotype of parthenocarpy and a complex of- most probably recessive - genes encoding the phenotype of functional sterility. These complexes are referred to as the "*pk*-complex" and the "*fs*-complex", respectively, and when combined in a single tomato plant, line or hybrid are referred to as the *pk,fs*-complex. The two parent plants or lines that contain these complexes and that are used to provide the seedless hybrids of the invention are referred to herein as the *"pk, fs-*parents".

The invention therefore in a first aspect relates to a method for producing a seedless tomato, a plant carrying seedless tomatoes or capable of carrying seedless tomatoes, or cultivation material for such a tomato plant such as seed, comprising
a. providing a first tomato plant that contains the *pk,fs*-complex (i.e. a first *pk,fs*-parent);
b. providing a second tomato plant that contains the *pk,fs*-complex (i.e. a second *pk,fs-*parent);
c. crossing the first and second tomato plant for the production of cultivation material, such as seed, which contains the *pk,fs*-complex;
d. optionally cultivating the cultivation material thus obtained into a tomato plant capable of carrying seedless tomatoes;
e. optionally growing said tomato plant until it carries the seedless tomatoes, and harvesting the seedless tomatoes thus obtained. More specifically, the invention relates to such a method comprising
a. providing a first tomato plant that contains the *pk,fs*-complex and that is further homozygote dominant with respect to at least one property desired for breeding tomatoes;
b. providing a second tomato plant that contains the *pk,fs-*complex and that is further homozygote recessive with regard to at least one property desired for the breeding of tomatoes;
c. crossing the first and second tomato plant for the production of cultivation material, such as seed, which contains the *pk,fs*-complex and that is further heterozygote with regard to at least one property desired for the breeding of tomatoes;
d. optionally cultivating the cultivation material thus obtained into a tomato plant capable of carrying seedless tomatoes;
e. optionally growing said tomato plant until it carries the seedless tomatoes, and harvesting the seedless tomatoes thus obtained. In the above methods, step c preferably comprises the following steps:
c1. providing pollen derived from the first or second tomato plant, respectively,
c2. fertilizing the (pre-embryo's of the) second or first tomato plant respectively, with the pollen obtained in step cl.

The cultivation material obtained in step c) is preferably hybrid seed or seedlings obtained from such seed, so that (also) the tomato plant obtained from said cultivation material in step d) is a hybrid tomato plant, with the ensuing advantages known per se associated with the known effect of heterosis, increased vigor, etc.. Hereinbelow, under the term "hybrid", both this hybrid cultivation material as well as these hybrid tomato plants are comprised, regardless of whether the latter (already) carry the seedless tomatoes according to the invention.

By the combination of the recessive phenotype of parthenocarpy and the recessive phenotype of functional sterility, these hybrids do not self-pollinate. Nevertheless, the hybrids will form the desired, regularly formed seedless fruits, without the need of (self)-pollination/fertilization of the (pre-embryo's on the) hybrid plant (which according to the invention is neither carried out nor required).

The first and second tomato plant provided in step a) and b) respectively prefererably belong to stable inbred lines, and consequently are herein denoted as the *pk,fs*-parent lines. These parent lines are preferably genetically stable, such as obtained by cultivation/inbreeding over several generations, as further described below.

In the above method, the first tomato plant can serve as the "father" (i.e. the plant from which the pollen is obtained) and the second tomato plant can serve as the "mother" (i.e. the plant of which the pistil/the pre-embryo's are fertilized with the pollen of the father), or vice versa. This is not essential for the method of the invention, as in both cases a hybrid with the desired *pk,fs*-complex, and therefore with the desired stable seedless fenotype, will be obtained.

In another aspect, the invention relates to a method for providing a hybrid seedless tomato plant or seed or other cultivating material for such a plant, comprising at least a step of pollinating a first seedless tomato plant in which the seedless fenotype is due to the presence of an essentially recessive complex of at least 4, preferably at least 5, more preferably at least 6 genetic factors, of which at least two factors provide said first seedless tomato plant with some phenotypical properties of partenocarpy and at least two factors provide said first seedless tomato plant with some phenotypical properties of functional sterility; with pollen obtained from a second seedless tomato plant in which the seedless fenotype is due to the presence of an essentially recessive complex of at least 4, preferably at least 5, more preferably at least 6 genetic factors, of which at least two factors provide the tomato plant with some phenotypical properties of partenocarpy and at least two factors provide the tomato plant with some phenotypical properties of functional sterility, and in which said phenotypical properties of partenocarpy and functional sterility contribute to the overall seedless phenotype of the tomato plant.

The step of pollinating the first seedless tomato plant with pollen from the second tomato plant involves the steps of opening - preferably manually- the closed off pollen tube of the second tomato plant; removing - preferably manually- pollen from the pollen tube of the second tomato plant: and applying -preferably manually - said pollen to the pistil of the first tomato plant.

The first and second tomato seedless tomato plant preferably belong to inbred lines, more preferably to two different inbred lines.

This aspect of the invention may optionally comprise the further steps of allowing the first seedless tomato plant thus fertilized to form fruits that contain hybrid seed and harvesting said hybrid seed from the fruits; and may also optionally comprise the yet further steps of cultivating a generation of seedless hybrid tomato plants from said hybrid seed, allowing said seedless hybrid tomato plants to form fruits (i.e. seedless tomatoes), and harvesting said seedless tomatoes.

Yet other aspects of the invention relate to the hybrid seed thus obtained, or optionally to other cultivating material obtained from said seed such as seedlings; to the seedless hybrid tomato plants thus obtained; and to the seedless tomatoes obtained as fruits from said seedless hybrid tomato plants.

Yet another aspect of the invention relates to processed products, in particular processed food products, obtained from or using the seedless tomatoes thus obtained.In the above aspects of the invention, two essentially sterile as well as seedless parents are made - through human intervention - to form hybrid seed.

In a further aspect, the invention relates to a seedless tomato plant, in which the seedless fenotype is due to the presence of an essentially recessive complex of at least 4, preferably at least 5, more preferably at least 6 genetic factors, of which at least two provide the tomato plant with some phenotypical properties of partenocarpy and at least two provide the tomato plant with some phenotypical properties of functional sterility, and in which said phenotypical properties of partenocarpy and functional sterility contribute to the overall seedless phenotype of the tomato plant.

In one specific aspect of the invention, this seedless tomato plant belongs to an inbred line, and can be used as a parent line to provide hybrid offspring. In another aspect, this seedless tomato plant is a hybrid obtained from two such, different inbred seedless parent lines, and may be used to grow or obtain seedless tomatoes.

In yet another aspect, the invention relates to the use of a seedless tomato plant in which the seedless fenotype is due to the presence of an essentially recessive complex of at least 4, preferably at least 5, more preferably at least 6 genetic factors, of which at least two provide the tomato plant with some phenotypical properties of partenocarpy and at least two provide the tomato plant with some phenotypical properties of functional sterility, in which said phenotypical properties of partenocarpy and functional sterility contribute to the overall seedless phenotype of the tomato plant; as a parent plant for obtaining seedless hybrid offspring.

This aspect of the invention can be carried out either by pollinating said seedless tomato plant with pollen obtained from another seedless tomato plant in which the seedless fenotype is due to the presence of an essentially recessive complex of at least 4, preferably at least 5, more preferably at least 6 genetic factors, of which at least two provide the tomato plant with some phenotypical properties of partenocarpy and at least two provide the tomato plant with some phenotypical properties of functional sterility, and in which said phenotypical properties of partenocarpy and functional sterility contribute to the overall seedless phenotype of the tomato plant; and/or by using pollen from said seedless tomato plant to pollinate another seedless tomato plant in which the seedless fenotype is due to the presence of an essentially recessive complex of at least 4, preferably at least 5, more preferably at least 6 genetic factors, of which at least two provide the tomato plant with some phenotypical properties of partenocarpy and at least two provide the tomato plant with some phenotypical properties of functional sterility, and in which said phenotypical properties of partenocarpy and functional sterility contribute to the overall seedless phenotype of the tomato plant

In this aspect of the invention, two essentially sterile as well as seedless parents are made - through human intervention - to form hybrid seed. The step of pollinating the first seedless tomato plant with pollen from the second tomato plant involves the steps of opening - preferably manually- the closed off pollen tube of the second tomato plant; removing - preferably manually- pollen from the pollen tube of the second tomato plant and applying -preferably manually -said pollen to the pistil of the first tomato plant.

Another aspect of the invention relates to a method for maintaining seedless tomato plant, in particular for maintaining an inbred line of seedless tomato plants, and/or for obtaining seed or other cultivating material for such a plant or line, comprising at least a step of pollinating a seedless tomato plant in which the seedless fenotype is due to the presence of an essentially recessive complex of at least 4, preferably at least 5, more preferably at least 6 genetic factors, of which at least two factors provide said first seedless tomato plant with some phenotypical properties of partenocarpy and at least two factors provide said first seedless tomato plant with some phenotypical properties of functional sterility; with pollen obtained from the same plant, or with pollen from another plant belonging to the same inbred line.

The step of pollinating the seedless tomato plant with its pollen involves the steps of opening - preferably manually- its closed off pollen tube; removing - preferably manually- pollen from the pollen tube; and applying -preferably manually -said pollen to the pistil of the tomato plant.

This aspect of the invention may optionally comprise the further steps of allowing the seedless tomato plant thus fertilized to form fruits that contain seed and harvesting said seed from the fruits; and may also optionally comprise the yet further steps of cultivating a further generation of seedless tomato plants from said seed.

Further aspects of the invention will become clear from the description hereinbelow.

According to one embodiment of the invention, the essentially recessive complex of at least 4, preferably at least 5, more preferably at least 6 genetic factors, comprises at least the genes *pat-2 and ps-2,* as well as at least 2, preferably at least 3, more preferably at least 4 additional genes, more preferably so that the *pat-2* gene and at least one, preferably at least two, additional genetic factors provide the tomato plant with some phenotypical properties of partenocarpy, and so that *the ps-2* gene and at least one, preferably at least two additional genetic factors provide the tomato plant with some phenotypical properties of functional sterility, in which said phenotypical properties of partenocarpy and functional sterility contribute to the overall seedless phenotype of the tomato plant.

### Brief description of the Figure and definitions:

The invention will be illustrated by reference to the enclosed Figure, which shows the cross between the *pk,fs*-parent lines, and also schematically illustrates a general method for developing the *pk,fs*-parent line(s), i.e. by means of a preferred example of this method involving generating an F6 generation starting from a known seedless tomato line, i.c. the *pat-2, ps-2* line of Georgiev. The tomato plants shown in the Figure are as follows:

### 1) Original seedless parent:

Tomato plant used as the first parent (line) to obtain the *fs,pk*-parents of the invention. The original seedless parent can be used as the male or female.
The original seedless parent will have a seedless fenotype, i.e. a combination of a partenocarpic fenotype and a functionally sterile fenotype. Preferably, the partenocarpic fenotype of the original seedless parent is due to the presence of homozygote recessive *pat-2* gene *(pat-2, pat-2),* but it may also be due to the presence of the *pat* gene, the *pat-3/pat-4* genes, *the pat-5* gene, or another gene.
Preferably, the functionally sterile fenotype of the original seedless parent is due to the presence of the homozygote recessive *ps-2* gene *(ps-2, ps-2).*
However, the invention is not limited thereto, and also other seedless lines known per se can be used.
The original seedless parent can be obtained in a manner known per se, for instance starting from a known tomato line that contains one of the known partenocarpy genes and/or that has a partenocarpic fenotype, such as
*Severianin* or the *pat-2* lines described by Georgiev, Michailov and Popova in the references from the Eucarpia Tomato Working Group, or by Philouze in the Agronomie reference, or partenocarpic lines containing the *pat, pat-3, pat-4, or pat-5* genes, such as those described in the art; and from a known tomato line with a functionally sterile fenotype, such as the *ps-2* containing lines described by Georgiev and Atanassova in the reference from the
Eucarpia Tomato Working Group. These are then crossed and selected to provide the original seedless parent, usually in an F2, F3 or further generation, and/or until a stable seedless line suitable for use as an original seedless parent is obtained.
Alternatively, and preferably, a seedless line known per se is used as the original seedless parent, such as the ps-2, pat-2 line described by Georgiev et al., in the Eucarpia Tomato reference. Again, such a line can also be obtained *de novo* from a source of a pat-2 gene and a ps-2 gene, i.e. analogous to the methods described in the above art.

### 2) Original non-seedless parent:

Tomato plant used as the second parent (line) to obtain the *pk,fs*-parents of the invention, by crossing with the original non-seedless parent. The original seedless parent can be used as the male or female.
The original non-seedless parent has a non-seedless fenotype, and preferably also not a partenocarpic or functionally sterile fenotype (although the use of a tomato plant or line with either a partenocarpic or functionally sterile fenotype is not excluded, and -for the purposes of description of the original non-seedless line - are included within the term "non seedless" and "original non-seedless parent").
As such, any known tomato plant, line or hybrid known per se can be used, including but not limited to all lines or hybrids that are commercially available, such as those mentioned in for instance the B-list of NAKG, the Netherlands, as well as all lines proprietary to plant breeders.
Preferably, a tomato plant belonging to an inbred line or a hybrid tomato is used.
Types of tomato which were found to be particularly suited for use as a non-seedless parent in the invention were lines belonging to the cherry tomatoes, determinant tomato lines, and/or tomato lines that carry genes for cold resistance such as *Lucia* or *Havana.*
Some other non-limiting examples of suitable, commerially available lines include *Daniëlle* and *M H One,* although commercial breeders may, and usually will, use their own proprietary lines.
Other suitable lines can be determined by the skilled person on the basis of the teaching given hereinbelow.

### 3) pk,fs-parent:

Tomato plant that contains the *pk,fs* complex as defined herein, and that can be used to provide a seedless hybrid by crossing with another *pk,fs* parent. Usually, the *pk,fs* parent will be in the form of a line, more particularly an inbred line.
*The pk,fs* parents can be obtained - by means of human intervention as described below- from the original seedless parent and the original non seedless parent (optionally though additional backcrosses), or starting from an already established *pk,fs* parent. It is envisaged that the present invention will usually be commercialized either by marketing the *pk,fs* parent line, or by marketing hybrid seed obtained from two *pk,fs* parent.

### 4) Seedless hybrid:

Hybrid tomato plant, obtained from two different *pk,fs*-parents by human intervention as described below.
The seedless hybrid is the plant that will be used by the grower/producer to grow and produce the seedless tomatoes. The seedless hybrid will have inherited the *pk,fs*-complex from its two *pk,fs*-parents, and will therefor show the seedless phenotype, i.e. grow fruits from its flowers without prior pollination.

### Method for obtaining the pk,fs parent lines.

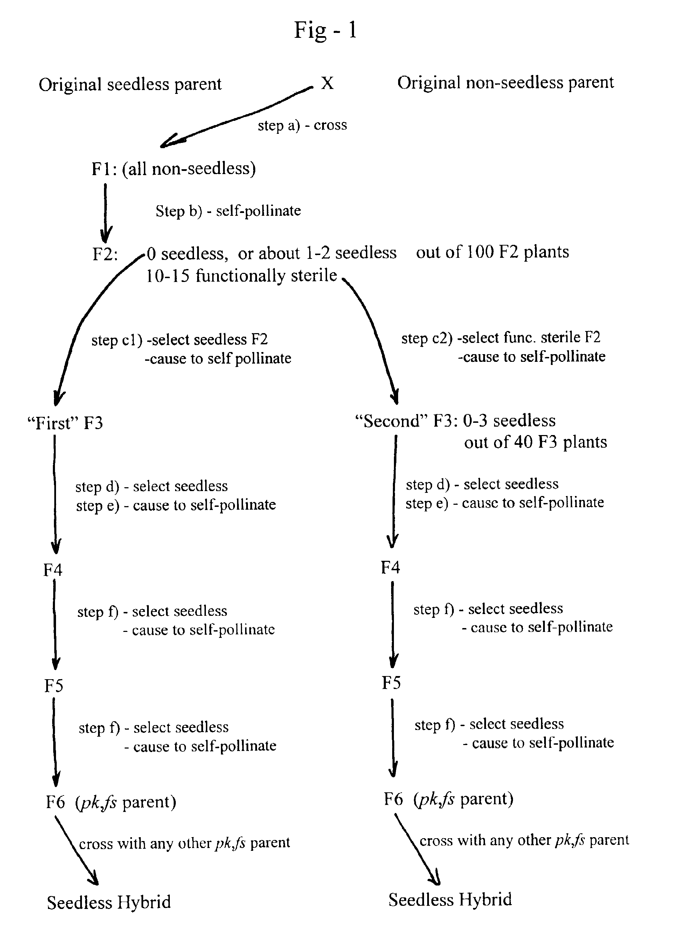

One method for obtaining the *pk,fs* parents is shown in Figure 1 and generally comprises the following steps, further discussed below:
a. Crossing an "original seedless parent" as defined herein with a "original non seedless parent" as defined herein to provide a non-seedless F1 generation;
b. Self-pollinating the F1 generation thus obtained to provide a further generation, herein referred to as the F2 generation.
c1. Selecting any plant(s) of the F2 thus obtained with a seedless fenotype and causing these seedless plants to self-pollinate, to provide a first F3 generation; as well as
c2. Selecting any plants of the F2 thus obtained with a functionally sterile fenotype, and causing these plants to self-pollinate in order to provide a second F3 generation;
d. Selecting any plants of the first or second F3 generation with a seedless fenotype;
e. Causing the plants of the first or second F3 generation with a seedless fenotype to self-pollinate in order to provide an F4 generation
f. Causing the plants of the F4 generation thus obtained which have a seedless fenotype to self-pollinate in order to provide an F5 generation; and optionally causing the plants of the F5 generation thus obtained which have a seedless fenotype to self-pollinate in order to provide an F6 generation.

Usually, by the F5 generation, and in particular by the F6 generation obtained in step e), the *pk,fs* complex in the plants thus obtained will have sufficiently stabilized, i.e. become "fixated", for the tomato plant to be used as *a pk,fs* parent in the invention, or to be used as a starting plant or line for obtaining other (lines of *pk,fs* parents, i.e. by crossing in further properties or by means of backcrosses.

As the F3, F4, F5, F6 and further generations have a seedless fenotype - or in the case of the "second F3 generation" at least a functionally sterile fenotype-obtaining the F4, F5, F6 and further generations, as well as maintaining *the pk,fs* parent lines for production of the seedless hybrids, will require human intervention as defined hereinbelow. This requirement for human intervention to provide a further generation is also generally referred to herein as *"causing self-pollination".*

It has been found that the F2 will usually only contain at most about 1 or 2 seedless plants in 100 F2 plants, but may also provide no seedless plants, depending upon the orginal seedless parent and in particular the orginal non-seedless parent used. Even when seedless F2 plants are obtained, it was found that the amount thereof (i.e. 1-5%) is significantly less than the 8,25 % (i.e. 1 plant out of 16) that was to be expected according to Mendelian principles if the presence of double recessive *pat-2 and ps-2* genes by itself were sufficient to provide a seedless phenotype. This shows that the cross with the original non-seedless parent apparently introduces some - probably dominant - genes, alleles or other genetic factors that negatively influence the occurrance of the seedlees phenotype in the F2.

The F2 will usually contain some functionally sterile plants, i.e. usually about 10-15 plants out of 100, again depending upon the orginal seedless parent and in particular the orginal non-seedless parent. This again is less than the 25% that was to be expected according to Mendelian principles if the presence of double recessive *ps-2* by itself were sufficient to provide a functionally sterile phenotype. This shows that also the presence of the desired functionally sterile fenotype is determined by a complex of genetic factors.

The seedless plants from the F2 are selected and caused to self-pollinate, so as to provide an F3, herein referred to as the "first F3 generation". It was found that, despite the seedless phenotype of the F2 plant, sometimes not all F3 plants thus obtained will show the seedless phenotype of the F2 plant, but can form 0-100%, and more often form only about 10-20% of the F3 plants. This again confirms that the seedless fenotype in this F2 plants is caused by a complex of genes (i.e. *the pk,fs* complex of the invention), and not by homozygote recessive *pat-2* and *ps-2* genes alone, as in the original seedless parent. It also shows that in the F2, the *pk,fs* complex is not fixated enough - i.e. genetically not homogenous enough- to provide completly seedless offspring. Because of this, the seedless F2 plants obtained as above are also not suited for use as a *pk,fs* parent in the invention.

The plants of the first F3 generation that show the seedless fenotype are selected, and caused to self-pollinate so as to provide an F4. Again, it is usually found that not all F4 plants obtained from the seedless F3 plants will show the seedless phenotype: the amount of seedless F4 plants may vary from 0-100%, and is usually about 10-20% of all F4 plants. Also, not all F4 plants are found to be seedless under all environmental conditions. This again shows that the *pk,fs* complex has not yet become sufficiently fixated in these F3 of F4 plants for them to be used as *pk,fs* parents in the invention.

The seedless plants from the F4 are then again caused to self-pollinate to provide an F5, and said the seedless plants from the F5 are caused to self-pollinate to produce an F6. Again, in the F5 and sometimes also in the F6, some non-seedless plants are obtained because the *pk,fs* complex was not yet sufficiently fixated in the seedless F4 or F5, respectively. Usually, by the F6 generation, the *pk,fs* complex in the seedless F6 plants will be sufficiently stable, so that all seedless F6 plants exclusively provide seedless F7 plants when caused to self-pollinate. This also is an indication that the F6 inbreds thus obtained can be used as *pk,fs* parents in the invention.

If the F7 still provides some non-seedless plants, the F7 may again be caused to self-pollinate to provide an F8, etc., until a generation is obtained in which the *pk,fs* complex is sufficiently fixated. However, this is usually not required and also not preferred. Also, if by the F9 and in particular by the F10 generation, the *pk,fs* complex has still not become sufficiently fixated, it will usually be assumed that this inbred line cannot be used as an *pk,fs* parent line in the invention.

Generally, only a small amount of the seedless F2 plants will "make it" to the F6, depending upon the orginal seedless parent but in particular the orginal non-seedless parent used. Also, in generating the F6, some selection pressure may be applied in order to test the stabilty and reliablity of the seedless phenotype under all environmental conditions. For instance, factors such as light, temperature can be used to "test" and/or fixate the stability of the seedless fenotype of the F3, F4, F5 or F6.

Besides the seedless F2 plants, also the F2 plants that only show a phenotype of functional sterility are caused to self-pollinate, so as to provide an F3 generation, herein referred to as the "second F3 generation". This second F3 generation will usually comprise essentially all functionally sterile plants, and may comprise some seedless plants, i.e. about 1-3 out of 40 F3 plants (which can be easily recognized and selected because they are the only ones in this second F3 that will grow fruits). If so, these seedless F3 plants are caused to self-pollinate to provide an F4, followed by an F5 and F6, and optionally an F7 and F8, etc., essentially as described for the first F3 generation.

Again, not all seedless plants of the second F3 generation will make it to the F6, again depending upon the orginal seedless parent and in particular the orginal non-seedless parent used.

In the above methodology, for a given combination of original seedless parent and original non-seedless parent, it is possible that no seedless plants are obtained in the F2, and only few functionally sterile plants. These functionally sterile F2 plants are then caused to self-pollinate. However, if in the F3 thus obtained, again no seedless plants are found, it will usually be assumed that this particular combination of original seedless parent and original non-seedless parent cannot be used to provide *a pk,fs* parent according to the invention. A possible explanation for this may be that the original non-seedless parent used did not contain all genetic factors (i.e. genes, alleles or other factors) necessary to "complete" the *pk,fs* complex, relative to the genes already present in the original seedless parent used.

However, the invention is not limited to any specific explanation or hypothesis as to what gene(s), alleles or other genetic factors make up the *pk,fs* complex according to the invention, nor to whether or not, and if so which or how many, genes or genetic factors (such as suppressors, modulators etc.) are necessary for the expression of the desired parthenocarpic fenotype and/or functionally sterile fenotype of the invention. However, as the present description teaches the artisan how the *pk,fs* parent(s) can be obtained starting from tomato plants or lines described in the art, such knowledge on the precise genetic make up of the *pk,fs* complex used in the invention is also not considered required for the skilled person to practice the invention (such lack of detailed knowledge of the precise genetic basis of a given phenotype is not unusual in the art).

This is also because the skilled person can easily determine -i.e. on the basis of the simple procedure outlined above- which non-seedless tomato plants or lines can be used in the invention to provide the additional genes, alleles or other factors necessary to "complete" the *pk,fs* complex, starting from a known seedless tomato plant or line, i.e. by determining the amount of seedless plants obtained in an F2 and/or F3 from a cross with the intended original seedless parent, as described above.

Also, it is not excluded that the genes, alleles or factors which form the *pk,fs* complex present in the *pk,fs* parents of the invention may also differ from one *pk,fs* parent plant or parent line to another, dependant upon which original seedless parent and non-seedless parent were used to obtain the *pk,fs* parent(s). It is therefore possible that included within the term *"pk,fs* complex" as used herein are in fact several different combinations of genes, alleles or other genetic factors, which all give the desired seedless phenotype in a manner sufficiently stable to reliably provide seedless offspring when combined with another *pk,fs* parent ( although if this is the case, such different complexes will usually have some, and probably most, of the most important genes, alleles or other genetic factors in common.)

Despite the above, it is assumed that the *"pk,fs"* complex comprises at least the partenocarpy gene or allele of the original seedless parent, combined with at least one further genetic genetic factor (i.e. gene, allele or other factor that may influence expression of parthenocarpy); and/or comprises at least the functional sterility gene of the original seedless parent also combined with at least one further genetic genetic factor (i.e. gene, allele or other factor that may influence expression of functional sterility), or a combination thereof.

Applicants research has also shown that the *pk,fs* complex probably consists of recessive genes, alleles or genetic factors, in that the phenotype of seedlessness is only uniformly inherited by hybrids obtained with another *pk,fs* parent. Because of this, for the purposes of this invention, the entire *pk,fs*-complex will be considered "essentially recessive"; and a cross with another *pk,fs* parent will usually be nessesary ensure that all genes, alleles or factors that make up the complex are uniformly inherited and expressed by the hybrid thus generated.

However, from applicants extensive research which has now spanned more than 10 years, as yet no satisfactory model has suggested itself that might clarify the number and/or the character of the genes, alleles or other genetic factors that are necessary to make up a *pk,fs* complex of the invention, or that might even explain the very low occurance or sometimes even the complete absence of seedless (or even functionally sterile) phenotypes in the F1, F2, F3 and even F4 obtained from the original seedless and non seedless parents. This shows that the factors determining the true seedless phenotype of the invention are much more intricate than suggested in the above prior art (i.e. not determined by a combination of (1 +1), (2 +1) or even (3+1) separate genes), and also explains why the prior art was not able to provide tomato plants or lines that can be used to produce seedless hybrids in a stable and reliable manner and under all environmental conditions.

It is possible that the *pk,fs* complex of the invention comprises one or more genes, alleles or other genetic factors (or a combination thereof) for either partenocarpy and/or for functional sterility that have not yet been described *per se* in the art. Similarly, it may also be possible the *pk,fs* complex of the invention comprises one or more of the known partenocarpy genes such as *pat-2, pat-3, pat-4* or *pat-5* (or a combination thereof), possibly in combination with one or more as yet unknown genes, alleles or other genetic factors that influence expression of partenocarpy; as well as one or more of the known functional sterility genes such as *ps, ex* or *short style,* again possibly in combination with one or more as yet unknown genes, alleles or other genetic factors that influence expression of functional sterility.

It is even possible that the genes, alleles or genetic factors involved in the properties of partenocarpy and functional sterility in fact somehow influence each others expression (i.e. in a manner not yet ascertained) so that the presence of one or more genes, alleles or genetic factors that determine partenocarpy are (also) required for good expression of functional sterility in the *pk,fs* parent lines and hybrids obtained therefrom, or visa versa, so as to provide together the desired seedless phenotype. In this specific explanation of the seedless fenotype of the invention, it can (also) not be completely excluded that the *pk,fs* complex of the invention comprises a single recessive gene for partenocarpy (such as *pat-2),* in combination with a complex of genes for functional sterility; or a single recessive gene for functional sterility (such as *ps-2),* in combination with a complex of genes for partenocarpy.

As to the scope of the present application, the *pat-2, ps-2* lines described by Georgiev are not included within the term *"pk,fs* complex" or *"pk,fs* parent", and also not within the scope of the invention.

For the purposes of further describing the *pk,fs* complex of the invention - and in particular for distinguishing the *pk,fs* plant from the seedless lines known in the art - the *pk,fs* complex may be considered to comprise a *"pk* part" (i.e. those genes, alleles or other inheritable factors that can influence the formation of the partenocarpic properties in the seedless phenotype) and a *"fs* part" (i.e. those genes, alleles or other inheritable factors that can influence the formation of the functioanlly sterile properties in the seedless phenotype). However, this manner of considering the *pk,fs* complex (i.e. as separate *pk* and *fs* parts) may not describe the actual situation *in planta,* because - as explained above - it cannot be excluded that some genetic factors from the *"pk"* part may also influence or be necessary for the *"fs* part", or visa versa; and there may also occur factors which influence or even are required for both the *"pk* part" and the *"fs* part", and consequently may be considered to belong to both.

When the *pk,fs* complex is defined in this way, the *"pk* part" of the *pk,fs* complex according to the invention comprises at least two, and preferably at least three genetic factors; and the *"fs* part" of the *pk,fs* complex also comprises at least two, and preferably at least three genetic factors (in which factors that influence both the partenocarpic properties as well as the functional sterility of the plant are counted as belonging both to the *"pk* part" as well as the *"fs* part". Similarly, a factor from the *"pk* part" that influences the *"fs* part", or visa versa, are also counted as belonging to both). In this definition, the "genetic factors" may be genes, alleles, multiple alleles or a combination thereof, as well as other factors that may influence expression, such as suppressors or promoter, co-dominance, incomplete dominance etc..

When this way of describing the *pk,fs* complex of the invention is applied analogously to the seedless lines known per se in the art, such as the *pat-2, ps-2* line of Georgiev, it will be clear that in these known plants, the *"pk* part" consists of a single factor (i.c. gene), and *the "fs* part" consists of a single factor (i.c. gene), which are also fully independant both in their interactions as well as their inheritance. Such plants or lines are not within the scope of the terms *"pk,fs* complex"or *"pk,fs* parent" as used herein. Similarly, by this definition, plants or lines in which the *"pk* part" consists of only a single genetic factor, and *the "fs* part" consists of only two genetic factors, or in which the *"pk* part" consists of only two factors, and the *"fs* part" consists of a single genetic factors, are generally not within the scope of the terms *"pk,fs* complex"or *"pk,fs* parent" as used herein, as these combinations are apparently sufficient to explain the inheritance of the pk,fs complex and/or seedless fenotype found in the invention.

By this definition, the art has so far not mentioned or suggested that the presence of a *pk,fs* complex in which the *"pk* part" consists of at least two, preferably at least three genetic factors, and also the *"fs* part" comprises at least two, preferably at least three genetic factors, is necessary to provide, in a stable and reliable manner, seedless hybrids that will form seedless tomatoes under all environmental, culturing or growing conditions. Also, none of the art describes tomato plants or lines that contain a *pk,fs* complex according to this definition.

Alternatively, the *pk,fs* complex could also be described as an essentially recessive complex of at least 4, preferably at least 5, more preferably at least 6 genetic factors, of which at least two factors provide said first seedless tomato plant with some phenotypical properties of partenocarpy and at least two factors provide said first seedless tomato plant with some phenotypical properties of functional sterility, and in which said phenotypical properties of partenocarpy and functional sterility contribute to the overall seedless phenotype of the plant. Again, tomato plants with a *pk,fs* complex according to this definition have not yet been described or provided by the art.

In one preferred embodiment, the original seedless parent is a *pat-2, ps-2* plant or line, such as the *pat-2, ps-2* line described by Georgiev. In this embodiment, it is assumed that - by the above definition- the *"pk* part" of the *pk,fs* parent derived from the original *pat-2, ps-2* seedless parent comprises the *pat-2* gene, combined with at least one additional genetic factor (i.e. a gene, allele or other factor that influences expression of parthenocarpy); and that the *"fs* part" comprises at least the *ps-2* gene, combined with at least one additional genetic factor (i.e. gene, allele or other factor that influences expression of functional sterility), or a combination thereof.

Alternatively, this embodiment of the *pk,fs* complex can also be described as an essentially recessive complex of at least 4, preferably at least 5, more preferably at least 6 genetic factors, comprises at least the genes *pat-2 and ps-2,* as well as at least 2, preferably at least 3, more preferably at least 4 additional genes, more preferably so that the *pat-2* gene and at least one, preferably at least two, additional genetic factors provide the tomato plant with some phenotypical properties of partenocarpy, and so that the *ps-2* gene and at least one, preferably at least two additional genetic factors provide the tomato plant with some phenotypical properties of functional sterility, and in which said phenotypical properties of partenocarpy and functional sterility contribute to the overall seedless phenotype of the tomato plant.

Above, the method for obtaining the *pk,fs* parent lines of the invention is specfically described starting from a known seedless line, such as a *pat-2, ps-2* line. However, based upon the present teaching, it will be clear to the skilled person that there will be possible equivalent methods for obtaining the *pk,fs* parent lines of the invention (i.e. lines with a "complete" *pk,fs* complex), starting from known lines that carry genes for partenocarpy and/or functional sterility, to which the additional genes necessary to "make up" the full *pk,fs* complex are then "added" from other tomato lines known per se. Such equivalent methods will generally be encompassed within the scope of the invention.

Besides providing the seedless hybrid with the *pk,fs* complex, the *pk,fs* parent lines are preferably such that they confer to their seedless hybrid offspring a number of predetermined properties which are desired for breeding, cultivating and/or growing tomato plants, and/or for the tomatoes produced by such plants. These properties are not specifically limited and for example comprise early development, increased growth, increased production, any form of the plant or fruit (including round, cylindrical, pear or cherry), the size or quality of the fruit, increased resistance against virusses or other diseases, increased cold resistance, long shelf life, etc., as will be clear to the person skilled in the art.

For this purpose, the *pk,fs* parent lines of the invention can be "designed" with the desired properties, for instance by appropriate choice of (the properties of) the original seedless and in particular non-seedless parents (as long as the original non-seedless parent provides the additional genes necessary to "complete" the *pk,fs* complex).

Alternatively, and usually, *pk,fs* parent lines with the desired properties are obtained starting from an already available/established *pk,fs* parent plant or line (such as those obtained as an F6 above), by introducing the desired property or properties into the *pk,fs* parent from a tomato line known per se, i.e. in a manner analogous to breeding techniques known per se.

In doing so, a tomato line known per se that carries the desired property (which will usually be a non-seedless tomato, but may also be a partenocarpic, functionally sterile or even other seedless line) is crossed or caused to cross with a *pk,fs* parent line to provide an F1 (which will usually not be seedless), which is then self-pollinated to provide an F2, from which the seedless plants can then be selected and again caused to self-pollinate into an F3, F4, F5 etc., until a stable inbred line that carries the desired properties as well as the *pk,fs* complex is obtained. In principle, this method can be essentially the same as the method described above, albeit that now a *pk,fs* parent used as a starting material instead of an "original seedless parent".

In this way, starting from one *pk,fs* parent obtained as decribed above, an entire range of *pk,fs* parent lines with desired properties can be obtained, which can then be crossed with each other - i.e. by means of human intervention - so as to provide the desired seedless hybrids. The use of a *pk,fs* parent as a starting material in this method will generally have the advantage that it will be ensured *ab initio* that all genes required to make up the *pk,fs* complex are present in starting material. ( Furthermore, although not preferred, the seedless hybrids of the invention can also be used as a source of the *pk,fs* complex for establishing new *pk,fs* parent lines, again essentially as described above).

In this embodiment, the above method for obtaining *the fs,pk* parents of the invention may therefore comprise the further steps of
g. causing the F6 *(pk,fs* parent) thus obtained to cross with a tomato plant that carries at least one desired property to generate an F1' generation; and
h. repeating steps b) to f) on the F1' thus obtained to generate an F6.

Another alternative is to carry out -i.e. as a part of the method described above- a backcross of the seedless plants of the (first or second) F3, F4 or F5 generation obtained from the original seedless and non-seedless parent, with a(nother) tomato plant or line that carries the desired property. The generation thus obtained is then used and further treated in the same way as the F1 generation obtained from the cross between the original seedless parent and the original non-seedless parent, i.e. via steps b) - f) as described above, to provide another *pk,fs* parent line. Optionally, such an F3, F4 or F5 backcross may be repeated more than once (i.e. on an F3, F4 or F5 obtained from a backcrossed F1), in order to introduce several desired properties from different tomato plants known per se into the final parent line. In this way, any desired properties can be introduced in the parent lines during and as part of the programme for developing the *pk,fs* parent, in less generations than would be required for introducing them into an established *pk,fs* parent line.

In this embodiment, the above method for obtaining the *pk,fs* parents may therefore comprise the further steps of
i. causing the plants of the first F3, second F3, F4 or F5 obtained after step c1), c2), e) or f) respectively to cross with a tomato plant that carries at least one desired property to generate an F1" generation; and
j. repeating steps b) to f) on the F1" thus obtained to generate an F6. In order to provide hybrids, and/or in order to promote heterosis and/or
vigourous growth, the *pk,fs* parent plants or lines are very preferably homozygote with regard to the one or more of the desired further properties, in which one parent plant will be homozygote dominant, and the other plant will be homozygote recessive, so that the resulting hybrid will be heterozygote with regard to said property, as is generally known per se for obtaining hybrid tomato plants by breeding.

The number, as well as the nature, of the properties for which the one parent will be homozygote recessive and the other will be homozygote dominant, will preferably be chosen such that the desired effect of heterosis is obtained in the resulting hybrid. For this purpose, for each of the several properties, the homozygote dominant and homozygote recessive genotypes can be distributed over the parent plants in any desired combination, which means that one parent plant can be homozygote dominant with respect to one desired property, and homozygote recessive with respect to another property, in which case the other parent plant will be homozygote recessive and homozygote dominant respectively, with respect to these properties. All this will be clear to the person skilled in the art of plant breeding.

It will also be clear to the skilled person that in producing the seedless hybrids of the invention, the uniformity of the hybrid (seed) will be essential for both the seed producer as well as the tomato grower, as this will not only ensure uniform quality of the cultivation material, but also of the hybrid plants and the eventually produced tomatoes. This is especially important for growing tomatoes on a large scalem, as well as for the final user, i.e. both industrial producers of tomato products as well as consumers.

As generally known in the art of plant breeding, the preferred manner for ensuring, in a repeatable and reliable manner, the uniformity of a hybrid is to use "pure" parent lines, obtained by inbreeding over several generations. The use of such inbred lines is generally also required for obtaining heterosis, for which the parent lines generally must be genetically "far apart". Usually, the above methods for obtaining the *pk,fs* parents as an inbred F6 (i.e. starting from either an original seedless parent, from another *pk,fs* parent line, or obtained by a F3, F4 or F5 backcross as described above) will at the same time ensure that the resulting *pk,fs* patent lines are also sufficiently pure for any other desired properties.

The presence of the *pk,fs* complex in the *pk,fs* parent lines also means that these lines are not capable of reproduction by themselves (i.e. of self-pollination). This means that human intervention - carried out essentially as described below for obtaining the seedless hybrids - required both for obtaining *pk,fs* parent lines (i.e by the methods described above), but also maintaining the *pk,fs* parent lines within the breeding station. Also, as the *pk,fs* parent lines themselves have the seedless phenotype, they will without such intervention also produce seedless tomatoes.

Thus, the *pk,fs* parent lines themselves, the above methods for obtaining and/or maintaining them; seed or other cultivating material for these parent lines, as well as the use of these parent lines in providing (seed for) seedless hybrid offspring, form further aspects of the invention, as do the seedless tomatoes produced by these *pk,fs* parents.

### Method for providing the seedless hybrids:

In order to provide the hybrids of the invention, two *pk,fs* parent lines must be crossed. However, as the *pk,fs* parent lines are sterile, and therefore cannot pollinate themselves or any other plant, this requires human intervention. (In essence, the invention comprises providing and maintaining two different sterile inbred parent lines, and than deriving seed as well as hybrid offspring from these two sterile as well as seedless lines. This cannot occur in nature. For this reason also, the *pk,fs* parent lines as well as the seedless hybrids generally cannot be considered a variety in the sense of the UPOV-treaty).

This human intervention generally comprises fertilizing the pre-embryo's of the flower of the first or second *pk,fs* parent, respectively, with pollen obtained from the second or first *pk,fs* parent, respectively, dependent upon the choice of the father and the mother.

Because the pollen tube of the father plant is closed, as a result of the functionally sterile phenotype, the pollen must be provided by opening said pollen tube, by machine or preferably by hand, in practice by cutting or scissoring the pollen tube.

Thereafter, the pollen is removed from the pollen tube, preferably again by hand, for instance by scraping, after which the pollen thus obtained is applied to the flower/pistil of the mother plant, again preferably by hand, such as by brushing or another suitable manner, such as spraying, to fertilize the (pre-embryo's of the) mother plant.

Theoretically, it is also possible - after opening the pollen tube, so that the enclosed pollen is released/accessible for spreading/distribution - to use other methods to transfer the pollen onto the pistil of the mother plant, such as using insects, especially when the pollen tube of the mother plants have not been opened or the stamen of the mother plant have been removed to prevent fertilization of the mother plant with its own pollen. Manual fertilization, as described hereinabove, is however very much preferred, both for reasons of efficiency, as well as to avoid fertilization/contamination of the mother. Moreover, manual pollination is a commonly used technique for obtaining hybrid tomatoes, to which for the purposes of the invention only the opening and scraping of the pollen tubes has to be added.

After pollination, the mother plant thus "fertilized" can be further cultivated until it carries tomatoes, which will contain the hybrid seed of the invention. This hybrid seed can then be collected in a manner known per se, optionally be processed further, as well as packaged for storage, transport or sale. Said hybrid seed, optionally in packaged form, forms an important aspect of the invention from a commercial point of view, as will be clear to the skilled person.

For obtaining the seedless hybrid tomato plant and the seedless tomatoes, the hybrid seeds can be sown in a manner known per se, or be made to germinate in another manner known per se in the art, and then be cultivated to tomato plants, which (can) carry the seedless tomatoes of the invention. As mentioned hereinabove no (self)-pollination/fertilization of the hybrid occurs; nevertheless a regularly formed fruit is produced on the not-fertilized hybrid. With the improved seedless hybrids of the invention, it is found that nearly all (i.e. more than 80%, and sometimes 95-99 % or more) of the flowers on the seedless hybrid will grow fruits (i.e. lead to "fruitset") as well as grow good fruits, and will do so reliably under all environmental or growing conditions. This makes the seedless hybrids of the invention a marked improvement over the seedless lines described in the art, which gave much poorer results, especially under conditions of bad light and/or low temperature.

The seedless tomatoes thus obtained can then be harvested and marketed and/or consumed as such, optionally after one or more further processing steps, such as sorting, washing or packaging.

The invention therefore in further aspects relates to cultivation material for tomatoes such as seed or seedlings (optionally in a container), as well as seedless tomatoes obtained and/or obtainable as described hereinabove, and/or suited for use in the method(s) described herein.

The seedless tomatoes according to the invention can also be processed further in a manner known per se to tomato products, in particular food products, which may or may not be in a form ready or suited for final use. In this respect, the tomatoes according to the invention have the advantage that they can be processed directly, without a further step for removing the seeds/pips in the production process.

The invention in a further aspect therefore relates to products, in particular food products, obtained from the seedless tomatoes according to the invention, as well as to a method for obtaining said food products, in which the tomatoes are processed to these products without a separate step for removing the seeds. Such a method can therefore -inter alia- comprise puréeing or mashing in another way of the tomatoes, optionally followed by incorporating or adding further desired ingredients, and packaging the tomato product thus obtained, without seeds or the residues thereof, in suitable containers for storage, transport or sale, in which said method does not comprise a step for removing any pips/seeds between the mashing of the tomatoes and the packaging of the product.

For such final use, another advantage of the seedless tomatoes of the invention is that they will have a higher content of fruit flesh (expressed as dry weight) compared to non-seedless tomatoes harvested at a corresponding time, i.e. 1% or more, based on total weight of the tomato (i.e. on average about 5,5 to 6,5 gram dry matter for the seedless tomatoes compared to about 4,5 to 5,5 gram dry matter for non-seedless tomatoes, on a total weight at harvest of about 110-120 gram). In terms of dry matter yield, this means an increase of at least about 20% ( in which furthermore the dry matter of the non-seedless tomatoes will still include the pips).

### EXPERIMENTAL PART.

### Introduction

Experiments were carried out using lines from a collection of homozygous breeding lines used for obtaining commercial hybrids.

In a first series, a small fruited, determinant line with the genes *ps2* and *path2* (received from Dr. Hristo Atanasov Georgiev) was used as the starting material. This line (indicated herein by the number 90173) proved to be seedless in growing conditions on the Canary Islands.

From this seedless line, further seedless lines are obtained by crossing with lines on the B list of NAKG, The Netherlands; with non listed hybrids or with open pollinated varieties of different countries (usually fresh market and industry tomato varieties with properties such as long shelf life, high exterior quality of the fruits disease resistances and so on.)

From these crosses F1 were grown, which were self-pollinated to provide an F2 which was selected for the properties fs, pk and/or fs. In the F3 the fspk plants were backcrossed with old or newly develop breeding lines.

In a second series, an advanced breeding lines with a functionally sterile fenotype (fs) and an advanced partenocarpic breeding line based upon *the pat-2* gene of Severyanin were used as the starting material. (As such, these *pat-2* lines generally have the problem of not uniform fruits in size and form and low till no seed in highly parthenocarpic lines.)

These fs and pk plants were crossed, and from these crosses F1 were grown, which were self-pollinated to provide an F2 which was selected for the properties fs, pk and/or fs. In the F3 the fspk plants were backcrossed with old or newly develop breeding lines.

### Initial experiments and comparative results.

From work with an fs line, it was found that ps2 itself is not sufficient for good and stable sterile plants in F2, F3 and higher F lines. Apparently some further, or modificator genes are necessary for full expression of this functional sterility.

In some cases small seedless fruits were found on fs-plants. Next generation growing gave the same result. This indicates that parthenocarpic fruit development is present although not fully, and probably due to genes different from path2. Also, the normal fs-plants do not develop fruits and flowers drop if not hand pollinated.

Working with path2 only it was noted specially in F3 that there are different levels of parthenocarpic development of the fruits. As a measurement, the number of fruits born on the castrated third cluster of a group of 20 plants was used. This indicates that more genes with parthenocarpic expression are required.

Working with fs,pk and pure breeding lines it was noted that in some F2 no fspk was found and in others for less numbers that the expected 1/16 based on two recessive genes, (ps2 path2). In F3 and following generations part if not all of fspk plants or lines were lost. Only a few succesfully reach F6.

This indicates that for seedless plants some gene or genes are missing for fully parthenocarpic expression under all environmental conditions. As low numbers or none fspk plants were found in F2, fs-plants were self-pollinated; sometimes fspk plants or even lines were found in F3. However, sometimes no F3 fspk plants or lines are found, and some of these plants or lines were lost in higher generation.

Because of the unstability of F2 fspk plants backcrosses on F3 lines or plants were carried out. In the F2 or F3 of this backcross no fspk plants or lines were found, depending on the recurrent parent.

Despite large numbers (200 F2 plants) and using all fs plants in F2 no fspk plants were found in F2 or F3 of one of the parents of Durinta F1 hybrid. Meanwhile one parent of Indian F1 gives in every F2 of a backcross fspk plants.

### Work on seedless lines.

Starting from the lines mentioned above, a number of different parents lines were developed that contained the *pk,fs* complex (indicated 1 to 9 and 1 to 16). These were crossed to provide a hybrid seedless F1, and seedlees fruitset and quality of fruit were determined. The results are as follows:

**TALBE 1 -**

| PERCENTAGE OF PARTHENOCARPIC FRUITSET AND PERCENTAGE OF FULL GROWN, GOOD FRUITS - ALL LINES USED ARE FSPK LINES | | |
|---|---|---|
| HYBRIDS | FRUITSET | GOOD FRUITS |
| 1 x 2 | 100 | 62 |
| 1 x 3 | 68 | 25 |
| 1 x 4 | 100 | 88 |
| 1 x 5 | 43 | 0 |
| 1 x 6 | 100 | 90 |
| 1 x 7 | 100 | 74 |
| 1 x 8 | 92 | 64 |
| 16 x 9 | 100 | 92 |
| 11 x 9 | 100 | 77 |
| 12 x 9 | 84 | 6 |
| 13 x 9 | 100 | 0 |
| 14 x 9 | 35 | 15 |
| 15 x 9 | 100 | 0 |
| 16 x 9 | 100 | 20 |

## Claims

1. Method for producing a seedless tomato, a plant carrying seedless tomatoes or capable of carrying seedless tomatoes, or cultivation material for such a tomato plant such as seed, comprising the steps of:
a. providing a first tomato plant that contains the *pk,fs*-complex (i.e. a first *pk,fs*-parent);
b. providing a second tomato plant that contains the *pk,fs*-complex (i.e. a second *pk,fs-*parent);
c. crossing the first and second tomato plant for the production of cultivation material, such as seed, which contains the *pk,fs*-complex;
d. optionally cultivating the cultivation material thus obtained into a tomato plant capable of carrying seedless tomatoes;
e. optionally growing said tomato plant until it carries the seedless tomatoes, and harvesting the seedless tomatoes thus obtained.

2. Method according to claim 1, comprising the steps of:
a. providing a first tomato plant that contains the *pk,fs*-complex and that is further homozygote dominant with respect to at least one property desired for breeding tomatoes;
b. providing a second tomato plant that contains the *pk,fs*-complex and that is further homozygote recessive with regard to at least one property desired for the breeding of tomatoes;
c. crossing the first and second tomato plant for the production of cultivation material, such as seed, which contains the *pk,fs*-complex and that is further heterozygote with regard to at least one property desired for the breeding of tomatoes;
d. optionally cultivating the cultivation material thus obtained into a tomato plant capable of carrying seedless tomatoes;
e. optionally growing said tomato plant until it carries the seedless tomatoes, and harvesting the seedless tomatoes thus obtained.

3. Method for providing a hybrid tomato plant capable of carrying seedless tomatoes, or seed or other cultivating material for such a hybrid tomato plant, comprising at least a step of pollinating a first seedless tomato plant in which the seedless fenotype is due to the presence of an essentially recessive complex of at least 4, preferably at least 5, more preferably at least 6 genetic factors, of which at least two factors provide said first seedless tomato plant with some phenotypical properties of partenocarpy and at least two factors provide said first seedless tomato plant with some phenotypical properties of functional sterility; with pollen obtained from a second seedless tomato plant in which the seedless fenotype is due to the presence of an essentially recessive complex of at least 4, preferably at least 5, more preferably at least 6 genetic factors, of which at least two factors provide the tomato plant with some phenotypical properties of partenocarpy and at least two factors provide the tomato plant with some phenotypical properties of functional sterility, and in which said phenotypical properties of partenocarpy and functional sterility contribute to the overall seedless phenotype of the tomato plant.

4. Method according to claim 3, in which the pollinating of the first seedless tomato plant with pollen from the second tomato plant involves the steps of opening - preferably manually- the closed off pollen tube of the second tomato plant; removing - preferably manually- pollen from the pollen tube of the second tomato plant: and/or of applying -preferably manually -said pollen to the pistil of the first tomato plant.

5. Method according to claim 3, in which the first and second tomato seedless tomato plant belong to inbred lines, preferably to two different inbred lines.

6. Method according to claim 3, comprising the further steps of allowing the first seedless tomato plant thus fertilized to form fruits that contain hybrid seed and harvesting said hybrid seed from the fruits; and optionally comprising the further steps of cultivating a generation of seedless hybrid tomato plants from said hybrid seed, allowing said seedless hybrid tomato plants to form fruits (i.e. seedless tomatoes), and harvesting said seedless tomatoes.

7. Seed for a hybrid tomato plant capable of carrying seedless tomatoes obtained via the method of claim 6, or cultivating material obtained from said seed such as seedlings.

8. Hybrid tomato plant capable of carrying seedless tomatoes, obtained via the method of claim 6.

9. Seedless tomato, obtained as fruit from the hybrid tomato plants of claim 8.

10. Processed products, in particular processed food products, obtained from or using, or containing, a seedless tomato according to claim 9.

11. Tomato plant with a seedless phenotype, in which said seedless fenotype is due to the presence of an essentially recessive complex of at least 4, preferably at least 5, more preferably at least 6 genetic factors, of which at least two provide the tomato plant with some phenotypical properties of partenocarpy and at least two provide the tomato plant with some phenotypical properties of functional sterility, and in which said phenotypical properties of partenocarpy and functional sterility contribute to the overall seedless phenotype of the tomato plant.

12. Tomato plant with a seedless fenotype according to claim 11, in which said plant belongs to an inbred line.

13. Tomato plant with a seedless fenotype according to claim 11, in which said plant is a hybrid.

14. Use of a tomato plant with a seedless phenotype, in which said seedless fenotype is due to the presence of an essentially recessive complex of at least 4, preferably at least 5, more preferably at least 6 genetic factors, of which at least two provide the tomato plant with some phenotypical properties of partenocarpy and at least two provide the tomato plant with some phenotypical properties of functional sterility, in which said phenotypical properties of partenocarpy and functional sterility contribute to the overall seedless phenotype of the tomato plant; as a parent plant for obtaining seedless hybrid offspring.

15. Method for maintaining a tomato plant with a seedless phenotype, in particular for maintaining an inbred line of such tomato plants, and/or for obtaining seed or other cultivating material for such a plant or line, comprising at least a step of pollinating a tomato plant with a seedless fenotype in which the seedless fenotype is due to the presence of an essentially recessive complex of at least 4, preferably at least 5, more preferably at least 6 genetic factors, of which at least two factors provide said first seedless tomato plant with some phenotypical properties of partenocarpy and at least two factors provide said first seedless tomato plant with some phenotypical properties of functional sterility; with pollen obtained from the same plant, or with pollen from another plant belonging to the same inbred line.

16. Method according to claim 15, in which the pollinating of the first seedless tomato plant with pollen from the second tomato plant involves the steps of opening - preferably manually- the closed off pollen tube of the second tomato plant; removing - preferably manually- pollen from the pollen tube of the second tomato plant: and/or of applying -preferably manually -said pollen to the pistil of the first tomato plant.

17. Method according to claim 15, comprising the further steps of allowing the seedless tomato plant thus fertilized to form fruits that contain seed and harvesting said seed from the fruits; optionally further comprising the steps of cultivating a further generation of seedless tomato plants from said seed.

18. Method according to claim 3, in which the essentially recessive complex of at least 4, preferably at least 5, more preferably at least 6 genetic factors, comprises at least the genes *pat-2* and *ps-2,* as well as at least 2, preferably at least 3, more preferably at least 4 additional genes, more preferably so that the *pat-2* gene and at least one, preferably at least two, additional genetic factors provide the tomato plant with some phenotypical properties of partenocarpy, and so that the *ps-2* gene and at least one, preferably at least two additional genetic factors provide the tomato plant with some phenotypical properties of functional sterility, in which said phenotypical properties of partenocarpy and functional sterility contribute to the overall seedless phenotype of the tomato plant.
